# EUROPÄISCHE PATENTSCHRIFT

(11) **EP 2 546 227 B1**
(45) Veröffentlichungstag und Bekanntmachung des Hinweises auf die Patenterteilung: **04.05.2016**
(21) Anmeldenummer: 11005653.8
(22) Anmeldetag: 11.07.2011
(51) Int. Cl.: C07C 51/41, C07C 55/02, C07C 55/14, B01J 8/20

(54) **Verfahren zur Herstellung einer wässrigen Lösung von Salzen**
Method for producing an aqueous solution of salts
Procédé de fabrication d'une solution aqueuse de sels

(43) Veröffentlichungstag der Anmeldung: 16.01.2013
(73) Patentinhaber: Uhde Inventa-Fischer GmbH, 13509 Berlin (DE)
(72) Erfinder: Siebecke, Ekkehard, 10825 Berlin (DE); Bär, Mirko, 16547 Birkenwerder (DE); Raue, Eberhard, 16567 Schönfließ (DE)
(74) Vertreter: Pfenning, Meinig & Partner mbB

(56) Entgegenhaltungen:
- EP-A1- 0 000 158
- EP-A1- 0 122 005
- WO-A1-2010/115727
- DE-A1- 2 139 642
- Anonymous: "Chemisches Gleichgewicht", Wikipedia, der freien Enzyklopädie , Seiten 1-6, XP002666502, Gefunden im Internet: URL:http://de.wikipedia.org/wiki/Chemische s_Gleichgewicht [gefunden am 2012-01-12]

## Beschreibung

Die Erfindung betrifft ein Verfahren zur kontinuierlichen Herstellung von Lösung von Salzen, insbesondere zur Herstellung von Hexamethylendiaminadipat und eine Vorrichtung zur Durchführung eines derartigen Verfahrens. Gemäß der Erfindung wird vorgeschlagen, in einem ersten Schritt eine unterstöchiometrische Menge von Alkandiamin im Verhältnis zur Alkandicarbonsäure im Wasser umzusetzen und in einem nachfolgenden zweiten Schritt eine Nachdosierung mit Alkandiamin durchzuführen, wobei die Einstellung der stöchiometrischen Verhältnisse über eine pH-Wertmessung bei konstanter Temperatur erfolgt.

Verfahren zur kontinuierlichen Herstellung von wässrigen Lösungen von Salzen aus Alkandicarbonsäuren und Alkandiaminen, die insbesondere als Ausgangsprodukt zur Herstellung von Polyamiden benötigt werden, sind im Stand der Technik bekannt.

So beschreibt die EP 0 000 158 B1 ein kontinuierliches Verfahren zur Herstellung einer wässrigen Lösung von Salzen aus Alkandicarbonsäuren und Alkandiaminen. Die Herstellung der Salzlösung wird danach durch Umsetzen der entsprechenden Alkandicarbonsäuren mit den jeweiligen Alkandiaminen in einer wässrigen Lösung des jeweils herzustellenden Salzes erreicht, wobei man die wässrige Salzlösung im Kreis führt, zunächst Dicarbonsäuren mit Alkandiaminen im Unterschuss umsetzt und dann die restliche Menge Alkandiamin zugibt.

Ein weiteres Verfahren zur Synthese von Hexamethylendiaminadipat aus Adipinsäure und Hexamethylendiamin zur Herstellung von Nylon 66 ist aus der EP 0 122 005 B1 bekannt. Bei diesem Verfahren wird zuerst eine wässrige Lösung des Salzes hergestellt und dann in einem nachfolgenden Verfahrensschritt durch Verdampfung eine Konzentrierung der Lösung erreicht. Letztlich wird dann bei diesem Verfahren offensichtlich, um die Verluste des Hexamethylendiamins beim Verdampfen auszugleichen, eine Nachdosierung des Hexamethylendiamins bis zu einem äquimolaren Verhältnis von Hexamethylendiamin zu Adipinsäure durchgeführt.

Es hat sich hier aber gezeigt, dass die Verfahren des Standes der Technik noch nicht in allen Punkten zu einem zufriedenstellenden Ergebnis führen. So ist insbesondere die Nachdosierung so wie sie in der EP 0 000 158 B1 vorgeschlagen wird, schwierig. Es hat sich herausgestellt, dass hierdurch keine exakte stöchiometrische Zusammensetzung realisierbar ist.

Auch weist das Verfahren, wie es in der EP 0 122 005 B1 beschrieben ist, noch Nachteile auf, da hierin die Alkandicarbonsäure als wässrige Lösung eingesetzt wird. Auch damit treten somit bei der Reaktion Verdampfungsverluste auf, die nicht mehr exakt steuerbar sind.

Ausgehend hiervon ist deshalb die Aufgabe der vorliegenden Erfindung, ein verbessertes kontinuierliches Verfahren zur Herstellung von wässrigen Lösungen von Salzen durch Umsetzung aus Alkandicarbonsäuren mit 6 bis 12 Kohlenstoffatomen und Alkandiamin mit 6 bis 12 Kohlenstoffatomen vorzuschlagen. Weiterhin ist es eine Aufgabe der vorliegenden Erfindung, eine entsprechende Vorrichtung hierzu anzugeben.

Die Aufgabe wird in Bezug auf das Verfahren durch die Merkmale des Patentanspruchs 1 und bezüglich der Vorrichtung durch die Merkmale des Anspruchs 13 gelöst. Die Unteransprüche zeigen vorteilhafte Weiterbildungen auf.

Erfindungsgemäß wird somit vorgeschlagen, die Salzlösung dadurch zu erhalten, dass man die Alkandicarbonsäure als Feststoff über mindestens eine erste Einspeisestelle in einen ersten Reaktor eindosiert und eine unterstöchiometrische Menge des unverdünnten Alkandiamins an mindestens einer zweiten Einspeisestelle sowie gleichzeitig Wasser an einer dritten Einspeisestelle des ersten Reaktors zudosiert. Die dadurch gebildete Salzlösung wird kontinuierlich in einen nachgeschalteten zweiten Reaktor überführt und im zweiten Reaktor erfolgt dann die Nachdosierung mit dem Alkandiamin. Erfindungswesentlich ist nun, dass die stöchiometrischen Verhältnisse im ersten und zweiten Reaktor über eine pH-Wertmessung bei konstanter Temperatur eingestellt werden.

Erfindungsgemäß sind der erste Reaktor und der zweite Reaktor Rührreaktoren.

Ein wesentlicher Vorteil des erfindungsgemäßen Verfahrens besteht darin, dass die Alkandicarbonsäure als Feststoff in den ersten Reaktor eingeführt wird und damit eine exakte Dosierung des Feststoffes möglich ist. Die Alkandicarbonsäure als Feststoff wird dabei bevorzugt über eine Dosiereinrichtung mit gravimetrischer Durchflussmessung in den ersten Reaktor zudosiert. Gemäß der Erfindung wird zusätzlich das Alkandiamin ebenfalls unverdünnt in den Reaktor eingeführt, so dass auch hier eine exakte Vorausbestimmung der für die benötigen stöchiometrischen Verhältnisse erforderlichen Menge exakt erfassbar ist. Wesentlich dabei ist weiterhin, dass dann in den ersten Reaktor mit dem unverdünnten Alkandiamin in der vorgegebenen Menge gleichzeitig Wasser zugeführt wird, um die entsprechende wässrige Lösung herzustellen. Die Einstellung des stöchiometrischen Verhältnisses erfolgt dabei über eine pH-Messung bei konstanter Temperatur. Die stöchiometrischen Verhältnisse von der Alkandicarbonsäure zum Alkandiamin im ersten Reaktor werden dabei so eingestellt, dass sie im Bereich von 1 : 0,80 bis 0,99, bevorzugt 1 : 0,85 bis 0,99 und besonders bevorzugt 1 : 0,95 bis 0,99 liegen. Der Druck, der im ersten Reaktor eingehalten werden soll, liegt im Bereich von 0,9 bis 1,2 bar (absolut) und die Temperatur im Bereich von 70 bis 120, insbesondere von 80 bis 95 °C, ganz besonders bevorzugt bei 90 °C.

Im ersten Reaktor wird die wässrige Lösung auf einer Konzentration an Salzen aus Alkandicarbonsäuren, Alkandiaminen von 50 bis 65 Gew.-%, besonders bevorzugt im Bereich von 63 Gew.-% gehalten.

Beim Verfahren nach der Erfindung wird in einem zweiten Schritt die so hergestellte Salzlösung kontinuierlich in einen nachfolgenden zweiten Reaktor überführt.

Im zweiten Reaktor wird nun so vorgegangen, dass dort eine Nachdosierung mit dem Alkandiamin vorgenommen wird, wobei auch hier wiederum das stöchiometrische Verhältnis über eine pH-Wertmessung, bei konstanter Temperatur, gesteuert wird.

Dadurch, dass nun, wie bei der Erfindung vorgeschlagen, zweistufig vorgegangen wird und in jeder einzelnen Stufe die Einstellung des molaren Verhältnisses von Alkandicarbonsäure zu Alkandiamin über eine pH-Wertmessung erfolgt, kann sichergestellt werden, dass beim erfindungsgemäßen Verfahren eine Salzlösung der Alkandicarbonsäure mit dem Alkandiamin im idealen Molverhältnissen erhalten wird, ohne dass dabei Verdampfungsverluste auftreten bzw. dass eine Überdosierung einer der Reaktionspartner erfolgt. Auch können etwaige Verdampfungsverluste mit einberechnet werden.

Beim erfindungsgemäßen Verfahren ist es weiterhin günstig, wenn sowohl im ersten Reaktor wie im zweiten Reaktor die Zuführung der Edukte, insbesondere die Zuführung der Alkandicarbonsäure in fester Form unter einer Schutzgasatmosphäre vorgenommen wird.

Das Verfahren nach der Erfindung kann dabei auch so ausgestaltet sein, dass das Wasser und das Inertgas für den ersten und/oder zweiten Reaktor im Kreislauf geführt wird.

Das Verfahren nach der Erfindung ist grundsätzlich auf alle Alkandicarbonsäuren mit 6 bis 12 Kohlenstoffatomen und alle Alkandiaminen mit 6 bis 12 Kohlenstoffatomen anwendbar. Bevorzugte Beispiele der Alkandicarbonsäuren sind Adipinsäure, Korksäure, Acelainsäure, Sebacinsäure, Decandisäure, Dodecandisäure sowie Mischungen hiervon. Beispiele für Alkandiamine sind Hexamethylendiamin, Octamethylendiamin, Decametyhlendiamin, Dodecanmethylendiamin, Bis(4-aminocyclohexyl)methan und Bis(4-aminocyclohexyl)propan-2,2.

Die Erfindung betrifft weiterhin eine Vorrichtung zur Durchführung des vorstehend beschriebenen Verfahrens.

Die Vorrichtung zeichnet sich insbesondere dadurch aus, dass zwei Reaktoren vorgesehen sind, die miteinander verbunden sind, wobei der erste Reaktor separate Einspeisestellen für die Edukte aufweist. Der erste Reaktor nach der Erfindung besitzt somit mindestens eine erste Einspeisestelle für die Alkandicarbonsäure, mindestens eine zweite Einspeisestelle für das Alkandiamin und mindestens eine dritte Einspeisestelle für das Wasser. Der zweite Reaktor weist zumindest ebenfalls eine Einspeisestelle für das Alkandiamin auf. Die Vorrichtung besitzt ferner noch Einrichtungen zur Messung des pH-Werts, der wässrigen Lösung von Salzen im ersten und/oder zweiten Reaktor und eine Steuerungseinheit, die anhand des gemessenen pH-Werts eine Nachdosierung des Alkandiamins im zweiten Reaktor ermöglicht.

Die Reaktoren sind erfindungsgemäß Rührreaktoren, die auch mit einem Heiz- oder Kühlmantel versehen sein können oder aber dass sie eine Heiz- oder Kühleinrichtung aufweisen.

Die Erfindung wird nachfolgend anhand der Figuren 1 bis 4 näher erläutert.
- Figur 1: zeigt die pH-Wert-Änderung in Abhängigkeit von der Konzentration einer AH-Salzlösung bei 90 °C,
- Figur 2: zeigt graphisch die pH-Wert-Änderung in Abhängigkeit von der Temperatur einer AH-Salzlösung von 63 %,
- Figur 3: zeigt die pH-Wert-Änderung bei Zugabe von HMD zu einer 63%igen AH-Salzlösung bei 90 °C und
- Figur 4: zeigt schematisch eine Vorrichtung zur Durchführung des erfindungsgemäßen Verfahrens.

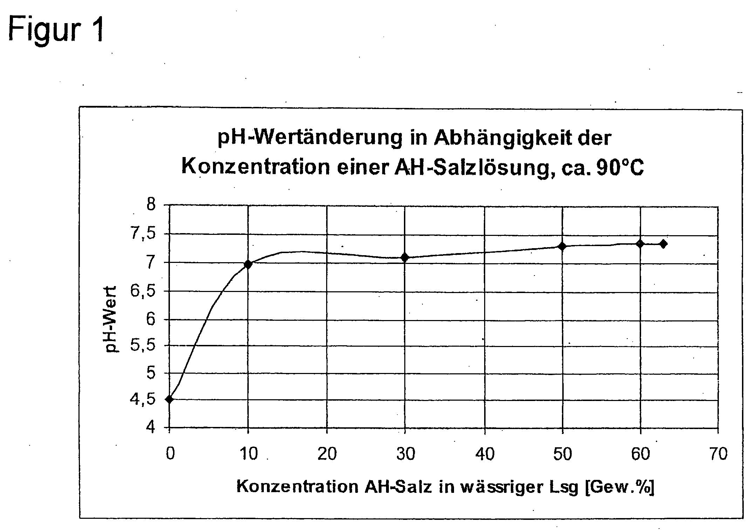

Die in den nachfolgenden Figuren 1 bis 3 wiedergegebenen Tabellen betreffen Beispiele, bei denen die Herstellung einer AH-Salzlösung (Hexamethylendiaminadipat) zu einer 63 %-igen wässrigen Lösung bei 90 °C aus fester Adipinsäure (ASS) sowie flüssiger Hexamethylendiamin-Lösung (HMD) durchgeführt worden ist.

Figur 1 zeigt nun die Abhängigkeit des pH-Werts von der Konzentration des AH-Salzes bei 90 °C. Wie aus der Figur 1 hervorgeht, ist im prozesstechnisch interessanten Bereich von 60 bis 63 % Gehalt an AH-Salz in der wässrigen Lösung bei 90 °C keine Abhängigkeit des pH-Wertes von der Konzentration an AH-Salz festgestellt worden.

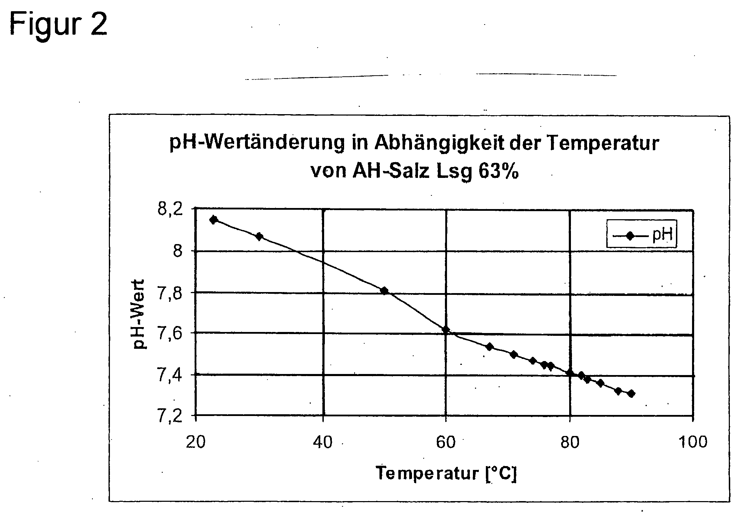

In Figur 2 wurde die Abhängigkeit des pH-Werts von der Temperatur untersucht.

Wie aus Figur 2 hervorgeht, zeigt sich eine starke Abhängigkeit des pH-Werts von der Temperatur von AH-Salzlösungen bei 63 %. Bei der Endtemperatur von 90 °C wurde ein pH-Wert der 63%igen wässrigen AH-Salzlösung von pH = 7,31 ermittelt. Im für den Prozess interessanten Temperaturbereich 70 bis 90 °C ändert sich der pH-Wert mit der Temperatur annähern linear. Pro °C Temperaturerhöhung wird ein Absinken des pH-Wertes um ca. 0,01 pH-Einheiten beobachtet.

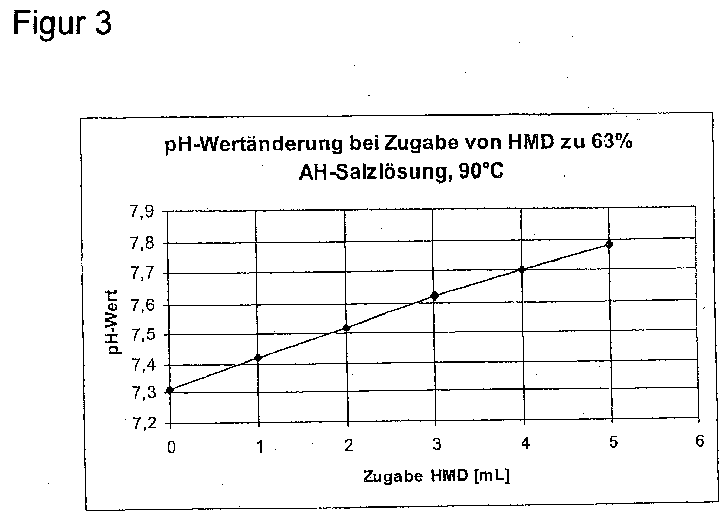

In Figur 3 ist nun die pH-Wert-Änderung bei Zugabe von HMD zu 63 %iger AH-Salzlösung bei 90 °C dargestellt. Wie aus Figur 3 hervorgeht, führt die Zugabe von 1 ml HMD-Lösung (entspricht ca. 7 mmol), d.h. einem Überschuss von HMD von 0,1 mol-% bezogen auf die 63%ige AH-Salzlösung zum Anstieg des pH-Wertes von ca. 0,01 pH-Einheiten.

Wie sich aus den vorstehend diskutierten Figuren 1 bis 3 ergibt, ist somit die Einstellung des molaren Verhältnisses über eine pH-Wertmessung unter der Maßgabe, dass die Temperatur konstant gehalten wird, in hinreichender Zuverlässigkeit möglich.

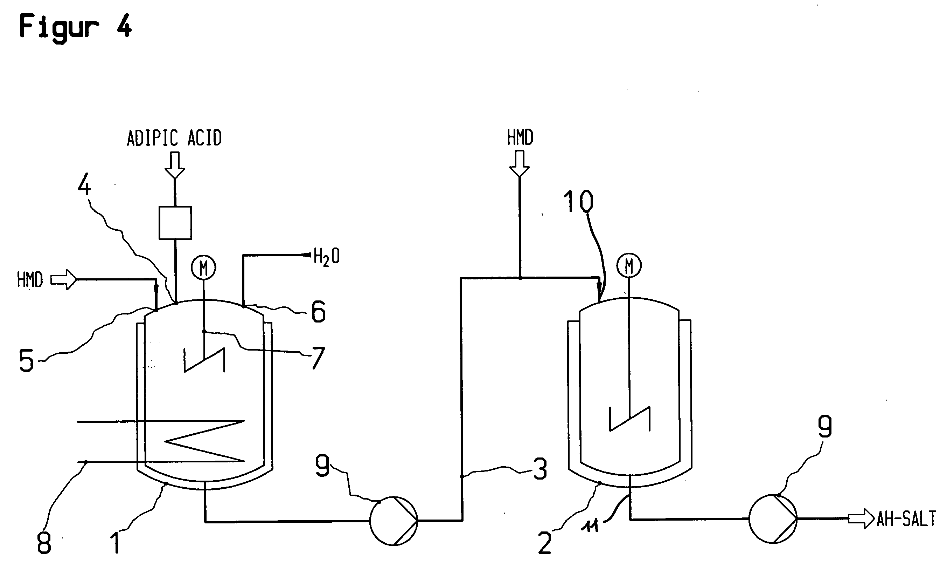

In Figur 4 ist nun schematisch eine erfindungsgemäße Vorrichtung zur Durchführung des Verfahrens dargestellt.

Die erfindungsgemäße Vorrichtung besteht im Ausführungsbeispiel nach der Figur 4 aus einem Rührreaktor 1 und einem Rührreaktor 2, der über eine Leitung 3 miteinander verbunden ist. Im Rührreaktor 1 ist zumindest eine erste Einspeisestelle 4 vorgesehen, die zur Zuführung fester pulverförmiger Adipinsäure dient. Die Adipinsäure (Adipic Acid) wird dabei aus einem nicht abgebildeten BigBag oder einem anderen geeigneten Transportbehältnis über eine entsprechende Dosiervorrichtung (ebenfalls nicht abgebildet) in den Rührreaktor 1 eingeführt. Der Rührreaktor 1 verfügt dann weiterhin über eine zweite Einspeisestelle 5, die zur Zuführung des Diamins, hier im Beispielsfall des Hexamethylendiamins (HMD), dient und eine weitere Einspeisestelle 6 zur Zufuhr von Wasser.

Der Rührreaktor nach der Erfindung ist, wie aus der Figur 4 hervorgeht, mit einem Rühraggregat 7 versehen und weist zudem eine Heiz- oder Kühlspirale 8 auf. Im Rührreaktor 1 wird die Zudosierung so wie vorstehend beschrieben, im unterstöchiometrischen Verhältnis vorgenommen und die Reaktionsführung so gesteuert, dass sich eine Konzentration von ca. 63 % im Rührreaktor 1 einstellt. Über die Leitung 3 und eine Pumpe 9 wird dann die Salzlösung in den Rührreaktor 2 überführt, wobei hier weiteres Hexamethylendiamin (HMD) über die Einspeisestelle 10 zudosiert wird. Die zudosierte Menge richtet sich dabei nach der pH-Wertmessung (nicht abgebildet) und wird automatisch ermittelt. Der Rührreaktor 2 ist dann am Boden mit einem Ablauf 11 versehen, so dass die entstehende AH-Salzlösung über eine Pumpe in einen ggf. vorhandenen Vorratsbehälter transportiert werden kann.

## Patentansprüche

1. Verfahren zur kontinuierlichen Herstellung einer wässrigen Lösung von Salzen durch Umsetzung aus Alkandicarbonsäuren mit 6 bis 12 Kohlenstoffatomen und Alkandiaminen mit 6 bis 12 Kohlenstoffatomen mit folgenden Schritten:
a) Eine definierte Menge Alkandicarbonsäure wird als Feststoff über mindestens eine erste Einspeisestelle in einen ersten Rührreaktor eindosiert und eine unterstöchiometrische Menge des unverdünnten Alkandiamins an mindestens einer zweiten Einspeisestelle, sowie gleichzeitig Wasser an mindestens einer dritten Einspeisestelle des ersten Rührreaktors, zur Herstellung einer wässrigen Salzlösung im Rührreaktor, zudosiert,
b) die gebildete Salzlösung wird kontinuierlich in einen nachgeschalteten zweiten Rührreaktor überführt und
c) es erfolgt eine Nachdosierung mit dem Alkandiamin im zweiten Rührreaktor, wobei die stöchiometrischen Verhältnisse im ersten und zweiten Rührreaktor durch eine pH-Wert-Messung bei konstanter Temperatur eingestellt werden.

2. Verfahren nach Anspruch 1,
**dadurch gekennzeichnet, dass** im ersten Rührreaktor ein pH-Wert von 6,0 bis 7 und im zweiten Rührreaktor ein pH-Wert von 6,8 bis 7,8, bevorzugt von 7,0 bis 7,4, eingehalten wird.

3. Verfahren nach Anspruch 1 oder 2,
**dadurch gekennzeichnet, dass** im ersten Rührreaktor ein stöchiometrisches Verhältnis von Alkandicarbonsäure zum Alkandiamin von 1 : 0,80 bis 0,99, bevorzugt 1 : 0,85 bis 0,99 und besonders bevorzugt 1 : 0,95 bis 0,99 eingehalten wird.

4. Verfahren nach einem der vorhergehenden Ansprüche,
**dadurch gekennzeichnet, dass** im ersten Rührreaktor die wässrige Lösung auf einer Konzentration an Salzen aus Alkandicarbonsäure und Alkandiaminen von 50 bis 65 Gew.-% gehalten wird.

5. Verfahren nach einem der vorhergehenden Ansprüche,
**dadurch gekennzeichnet, dass** die Umsetzung im ersten und zweiten Rührreaktor bei Temperaturen von 70 °C bis 120 °C, insbesondere von 80 °C bis 95 °C, besonders bevorzugt bei 90 °C, durchgeführt wird.

6. Verfahren nach einem der vorhergehenden Ansprüche,
**dadurch gekennzeichnet, dass** die im ersten Rührreaktor zudosierte Menge an Alkandiamin und Wasser in Abhängigkeit von der Menge an vorgelegter Alkandicarbonsäure bestimmt wird.

7. Verfahren nach einem der vorhergehenden Ansprüche,
**dadurch gekennzeichnet, dass** die Alkandicarbonsäure über eine Dosiereinrichtung mit gravimetrischer Durchflussmessung in den ersten Rührreaktor zudosiert wird.

8. Verfahren nach einem der vorhergehenden Ansprüche,
**dadurch gekennzeichnet, dass** dem ersten und/oder zweiten Rührreaktor über mindestens eine Zuleitung Inertgas zugeführt wird, um in den Rührreaktoren eine Inertgasatmosphäre zu gewährleisten.

9. Verfahren nach einem der vorhergehenden Ansprüche,
**dadurch gekennzeichnet, dass** das Wasser und das Inertgas für den ersten und/oder zweiten Rührreaktor im Kreislauf geführt wird.

10. Verfahren nach einem der vorhergehenden Ansprüche,
**dadurch gekennzeichnet, dass** die Alkandicarbon-säuren ausgewählt sind aus der Gruppe bestehend aus Adipinsäure, Korksäure, Azelainsäure, Sebacinsäure, Dekandisäure, Dodecandisäure sowie Mischungen hiervon.

11. Verfahren nach einem der vorhergehenden Ansprüche,
**dadurch gekennzeichnet, dass** das Alkandiamin ausgewählt ist aus der Gruppe bestehend aus Hexamethylendiamin, Octamethylendiamin, Deca-methylendiamin, Dodecamethylendiamin, Bis(4-aminocyclohexyl)methan und Bis(4-aminocyclo-hexyl)propan-2,2.

12. Vorrichtung zur kontinuierlichen Herstellung
einer wässrigen Lösung von Salzen durch Umsetzung aus Alkandicarbonsäuren mit 6 bis 12 Kohlenstoffatomen und Alkandiaminen mit 6 bis 12 Kohlenstoffatomen umfassend:
a) einen ersten Rührreaktor (1) für die Umsetzung der Alkandicarbonsäuren mit dem Alkandiamin, wobei der erste Rührreaktor (1) mindestens eine erste Einspeisestelle (4) für die Alkandicarbonsäuren, mindestens eine zweite Einspeisestelle (5) für das Alkandiamin und mindestens eine dritte Einspeisestelle (6) für das Wasser aufweist, wobei der erste Rührreaktor (1) eine Heiz- oder Kühleinrichtung (8) zur Einstellung einer konstanten Temperatur aufweist,
b) einen zweiten Rührreaktor (2), der mit dem ersten Rührreaktor (1) verbunden ist und der mindestens eine zusätzliche Einspeisestelle (10) für das Alkandiamin aufweist, wobei der zweite Rührreaktor (2) eine Heiz- oder Kühleinrichtung zur Einstellung einer konstanten Temperatur aufweist, sowie
c) Messvorrichtungen zur Messung des pH-Werts der wässrigen Lösung von Salzen im ersten und/oder zweiten Rührreaktor und
d) eine Steuerungseinheit, die anhand des gemessenen pH-Wertes eine Nachdosierung des Alkandiamins im zweiten Rührreaktor regelt.

13. Vorrichtung nach Anspruch 12, **dadurch gekennzeichnet, dass** der zweite Rührreaktor (2) mit einem Vorratsbehälter verbunden ist.

## Claims

1. Method for the continuous production of an aqueous solution of salts by conversion of alkane dicarboxylic acids with 6 to 12 carbon atoms and alkane diamines with 6 to 12 atoms, having the following steps:
a) a defined quantity of alkane dicarboxylic acid is metered as solid material into a first stirred reactor via at least one first feed point and a substoichiometric quantity of undiluted alkane diamine is metered in at at least a second feed point, and also, at the same time, water at at least one third feed point of the first stirred reactor, for the production of an aqueous salt solution in the stirred reactor,
b) the formed salt solution is transferred continuously into a subsequently connected second stirred reactor and
c) making-up with the alkane diamine is effected in the second stirred reactor, the stoichiometric ratios in the first and second stirred reactor being adjusted by a pH value measurement at a constant temperature.

2. Method according to claim 1,
**characterised in that**, in the first stirred reactor, a pH value of 6.0 to 7 is maintained and, in the second stirred reactor, a pH value of 6.8 to 7.8 preferably of 7.0 to 7.4.

3. Method according to claim 1 or 2,
**characterised in that**, in the first stirred reactor, a stoichiometric ratio of alkane dicarboxylic acid to alkane diamine of 1 : 0.80 to 0.99, preferably 1 : 0.85 to 0.99 and particularly preferred 1 : 0.95 to 0.99, is maintained.

4. Method according to one of the preceding claims,
**characterised in that**, in the first stirred reactor, the aqueous solution is maintained at a concentration of salts of alkane dicarboxylic acid and alkane diamines of 50 to 65% by weight.

5. Method according to one of the preceding claims,
**characterised in that** the conversion in the first and second stirred reactor is implemented at temperatures of 70°C to 120°C, in particular of 80°C to 95°C, particularly preferred at 90°C.

6. Method according to one of the preceding claims,
**characterised in that** the quantity of alkane diamine and water metered in in the first stirred reactor is determined as a function of the quantity of introduced alkane dicarboxylic acid.

7. Method according to one of the preceding claims, **characterised in that** the alkane dicarboxylic acid is metered into the first stirred reactor via a metering device with a gravimetric throughflow measurement.

8. Method according to one of the preceding claims,
**characterised in that** inert gas is supplied to the first and/or second stirred reactor via at least one supply line in order to ensure an inert gas atmosphere in the reactors.

9. Method according to one of the preceding claims,
**characterised in that** the water and the inert gas for the first and/or second stirred reactor are guided in a circulation.

10. Method according to one of the preceding claims,
**characterised in that** the alkane dicarboxylic acids are selected from the group consisting of adipic acid, octanedioic acid, azeleic acid, sebacic acid, decanedioic acid, dodecanedioic acid and also mixtures hereof.

11. Method according to one of the preceding claims,
**characterised in that** the alkane diamine is selected from the group consisting of hexamethylenediamine, octamethylenediamine, decamethylenediamine, dodecamethylenediamine, bis(4-aminocyclohexyl)methane and bis(4-aminocyclohexyl)propane-2,2.

12. Device for the continuous production of an aqueous solution of salts by conversion of alkane dicarboxylic acids with 6 to 12 carbon atoms and alkane diamines with 6 to 12 carbon atoms, comprising:
a) a first stirred reactor (1) for the conversion of the alkane dicarboxylic acids with the alkane diamine, the first stirred reactor (1) having at least one first feed point (4) for the alkane dicarboxylic acids, at least one second feed point (5) for the alkane diamine and at least one third feed point (6) for the water, the first stirred reactor (4) having a heating or cooling device (8) for adjusting a constant temperature
b) a second stirred reactor (2) which is connected to the first stirred reactor (1) and which has at least one additional feed point (10) for the alkane diamine, the second stirred reactor (2) having a heating or cooling device (8) for adjusting a constant temperature and also
c) measuring devices for measuring the pH value of the aqueous solution of salts in the first and/or second stirred reactor and
d) a control unit which controls making-up of the alkane diamine in the second reactor by means of the measured pH value.

13. Device according to claim 12, **characterised in that** the second stirred reactor (2) is connected to a storage container.

## Revendications

1. Procédé de préparation continue d'une solution aqueuse de sels par réaction d'acides alcanedicarboxyliques ayant 6 à 12 atomes de carbone et d'alcanediamines ayant 6 à 12 atomes de carbone, comportant les étapes suivantes :
a) une quantité définie d'un acide alcanedicarboxylique est, sous forme solide, introduite par au moins un premier point d'injection dans un premier réacteur agitateur, et on ajoute une quantité sous-stoechiométrique de l'alcanediamine non diluée par au moins un deuxième point d'injection, ainsi que, simultanément, de l'eau par au moins un troisième point d'injection du premier réacteur agitateur, pour préparer une solution aqueuse des sels dans le réacteur agitateur,
b) on transfère en continu la solution de sels formée dans un deuxième réacteur agitateur monté en aval, et
c) on dose encore l'alcanediamine dans le deuxième réacteur agitateur, les rapports stoechiométriques dans le premier et dans le deuxième réacteurs agitateurs étant ajustés par une mesure du pH à température constante.

2. Procédé selon la revendication 1, **caractérisé en ce qu'**on maintient dans le premier réacteur agitateur un pH de 6,0 à 7 et dans le deuxième réacteur agitateur un pH de 6,8 à 7,8, de préférence de 7,0 à 7,4.

3. Procédé selon la revendication 1 ou 2, **caractérisé en ce qu'**on maintient dans le premier réacteur agitateur un rapport stoechiométrique de l'acide alcanedicarboxylique à l'alcanediamine de 1:0,80 à 0,99, de préférence de 1:0,85 à 0,99 et d'une manière particulièrement préférée de 1:0,95 à 0,99.

4. Procédé selon l'une des revendications précédentes, **caractérisé en ce qu'**on maintient, dans la solution aqueuse dans le premier réacteur agitateur, une concentration en sels de l'acide alcanedicarboxylique et des alcanediamines de 50 à 65 % en poids.

5. Procédé selon l'une des revendications précédentes, **caractérisé en ce que** la réaction dans le premier et dans le deuxième réacteurs agitateurs est mise en oeuvre à des températures de 70°C à 120°C, en particulier de 80°C à 95°C, d'une manière particulièrement préférée à 90°C.

6. Procédé selon l'une des revendications précédentes, **caractérisé en ce qu'**on détermine en fonction de la quantité d'acide alcanedicarboxylique mis en place la quantité d'alcanediamine et d'eau dosée dans le premier réacteur agitateur.

7. Procédé selon l'une des revendications précédentes, **caractérisé en ce que** l'acide alcanedicarboxylique est dosé dans le premier réacteur agitateur par l'intermédiaire d'un dispositif doseur comportant une mesure gravimétrique du débit.

8. Procédé selon l'une des revendications précédentes, **caractérisé en ce qu'**un gaz inerte est envoyé dans le premier et/ou dans le deuxième réacteurs agitateurs par au moins une conduite d'amenée, pour garantir dans les réacteurs agitateurs une atmosphère d'un gaz inerte.

9. Procédé selon l'une des revendications précédentes, **caractérisé en ce que** l'eau et le gaz inerte destinés au premier et/ou au deuxième réacteurs agitateurs, sont amenés en circuit fermé.

10. Procédé selon l'une des revendications précédentes, **caractérisé en ce que** les acides alcanedicarboxyliques sont choisis dans le groupe consistant en l'acide adipique, l'acide subérique, l'acide azélaïque, l'acide sébacique, l'acide décanedioïque, l'acide dodécanedioïque, ainsi que les mélanges de ceux-ci.

11. Procédé selon l'une des revendications précédentes, **caractérisé en ce que** l'alcanediamine est choisie dans le groupe consistant en l'hexaméthylènediamine, l'octaméthylènediamine, la décaméthylènediamine, la dodécaméthylènediamine, le bis(4-aminocyclohexyl)méthane et le bis(4-aminocyclohexyl)propane-2,2.

12. Dispositif de préparation continue d'une solution aqueuse de sels par réaction d'acides alcanedicarboxyliques ayant 6 à 12 atomes de carbone et d'alcanediamines ayant 6 à 12 atomes de carbone, comprenant :
a) un premier réacteur agitateur (1) pour la réaction des acides alcanedicarboxyliques avec l'alcanediamine, le premier réacteur agitateur (1) comportant au moins un premier point d'injection (4) pour les acides alcanedicarboxyliques, au moins un deuxième point d'injection (5) pour l'alcanediamine et au moins un troisième point d'injection (6) pour l'eau, le premier réacteur agitateur (1) comportant un dispositif de chauffage ou de refroidissement (8) pour ajuster la température à une valeur constante,
b) un deuxième réacteur agitateur (2), qui est relié au premier réacteur agitateur (1) et qui comprend au moins un point d'injection supplémentaire (10) pour l'alcanediamine, le deuxième réacteur agitateur (2) comprenant un dispositif de chauffage ou de refroidissement pour ajuster la température à une valeur constante, ainsi que
c) des dispositifs de mesure pour mesurer le pH de la solution aqueuse de sels dans le premier et/ou dans le deuxième réacteurs agitateurs, et
d) une unité de commande, qui à l'aide du pH mesuré assure la régulation du dosage supplémentaire de l'alcanediamine dans le deuxième réacteur agitateur.

13. Dispositif selon la revendication 12, **caractérisé en ce que** le deuxième réacteur agitateur (2) est relié à un réservoir de stockage.
